# EUROPEAN PATENT SPECIFICATION

(11) **EP 4 363 532 B1**
(45) Date of publication and mention of the grant of the patent: **26.03.2025**
(21) Application number: 22758022.2
(22) Date of filing: 01.08.2022
(51) Int. Cl.: C10J 3/00, C12P 7/06

(54) **PROCESS AND APPARATUS FOR PRODUCING BIOETHANOL WITHOUT COEMISSIONS BY CONVERSION OF SYNGAS OBTAINED FROM THE THERMAL CONVERSION OF WASTE AT HIGH TEMPERATURE**
VERFAHREN UND VORRICHTUNG ZUR COEMISSIONSFREIEN HERSTELLUNG VON BIOETHANOL DURCH UMSETZUNG VON SYNGAS AUS DER THERMISCHEN UMSETZUNG VON ABFALLSTOFFEN BEI HOHER TEMPERATUR
PROCÉDÉ ET APPAREIL DE PRODUCTION DE BIOÉTHANOL SANS ÉMISSIONS DE COPAR CONVERSION DE GAZ DE SYNTHÈSE OBTENU À PARTIR DE LA CONVERSION THERMIQUE DE DÉCHETS À HAUTE TEMPÉRATURE

(30) Priority: 02.08.2021 IT 202100020819
(43) Date of publication of application: 08.05.2024
(73) Proprietor: NEXTCHEM TECH S.p.A., 00156 Rome (IT)
(72) Inventor: FOLGIERO, Pierroberto, 00186 Roma (IT); IAQUANIELLO, Gaetano, 00153 Roma (IT); RISPOLI, Giacomo, 00136 Roma (IT); SALLADINI, Annarita, 64026 Roseto degli Abruzzi (TE) (IT); BORGOGNA, Alessia, 65124 Pescara (IT)
(74) Representative: Sarpi, Maurizio
(86) International application number: PCT/IB2022/057126
(87) International publication number: WO 2023/012644

(56) References cited:
- CA-A1- 3 013 354
- CA-A1- 3 090 411
- US-B2- 9 926 196

## Description

The invention regards the field of processes and apparatuses suitable to convert RDF (Refuse Derived Fuel), MSW (Municipal Solid Waste) and similar materials such as plastic residues into products with higher added value by ensuring eco-compatibility in terms of absence of greenhouse gas emissions, and it specifically consists of a process and apparatus for producing bioethanol without CO₂ emissions by converting syngas obtained from the thermal conversion of waste at high temperature.

### State of the art

Global warming is forcing the European Union to actuate a strategy for eliminating greenhouse gas (GHG) emissions in Europe by 2050 by looking for alternatives to the use of natural gas or other hydrocarbons.

The conventional way to dispose of municipal, agricultural and industrial waste through incineration is also one of the main sources of greenhouse gas emissions, mainly CO₂, CH₄ and nitrogen oxides. In contrast, the waste can be used as a source of carbon and hydrogen by converting it into a syngas, a mixture of CO, CO₂ and H₂. The processes for such a conversion are known in the art.

For example, patent WO/2018/066013 describes a gasification process through the use of pure oxygen for the conversion of waste into syngas controlling the temperature along the vertical axis of the reactor.

Patent WO/2018/134853 describes a waste conversion process at high temperature for producing syngas, followed by the purification of the syngas and the regulation of the H₂/CO ratio for the purpose of producing methanol.

In both of the cited examples there is an excess of carbon in the matrix of the feed, with respect to the final product, which leaves the process mainly in the form of CO₂.

The further conversion of syngas into the desired products, in particular ethanol, can be performed through different ways.

Patent US9518237 describes a process for converting a syngas into oxygenated hydrocarbon products through anaerobic bioconversion; said hydrocarbon products are in particular ethanol, acetic acid, n-propanol and n-butanol.

Patent US2019/0078121 describes a different method for improving the CO/H₂ composition of the flow of gas available for supplying a microbial production of oxygenated compound.

Finally, in patent document US2019/0323042, the production of ethanol is obtained through anaerobic fermentation from a CO-rich syngas, but this implies that a fraction of carbon is lost as CO₂ whereas the production of CO₂ can only be reduced or avoided as the amount of available H₂ increases.

Therefore, the need remains for a process and apparatus with zero emissions, for producing chemical substances or biofuels such as ethanol, from the carbon contained in municipal solid waste (MSW), agricultural waste, or in derivatives thereof such as refuse derived fuel (RDF) and industrial waste such as non-recyclable plastic residues.

Refuse derived fuel (RDF) is a sort of fuel produced after the separation of the organic fraction, metals, paper and plastic.

Furthermore, there is a need for a process and apparatus which has zero emissions, in particular in relation to CO₂ emissions.

CA 3 090 411 A1, CA 3 013 354 A1 and US 9 926 196 B2 describe processes and apparatuses for improving carbon capture and fermentation efficiency in ethanol production by utilising gasification-derived substrates, with emphasis on optimising hydrogen and carbon monoxide ratios through electrolysis and partial oxidation to reduce CO₂ emissions and enhance carbon conversion.

### Aim of the invention

The aim of the invention is to overcome the limits of the prior art by providing a process and apparatus which are compatible with the guidelines of the regulations aiming at reducing greenhouse gas emissions in the environment to zero and suitable for the conversion of municipal waste, together with other industrial waste, such as non-recyclable plastics, into bioethanol.

### Provided solution

The provided solution is a process, and the related apparatus, in which the CO₂ emissions are avoided due to the synergistic effect of a fermentation step and a methanation step, also by considering that it is well known that the gasification of such feed implies a greater production of CO and CO₂, so as to produce a syngas which is enriched with extra hydrogen from electrolytic processes and thus maximizing the biological fermentation process for producing ethanol.

### List of figures

A better understanding of the invention will be had by means of the following detailed description and with reference to the accompanying drawings, illustrating a preferred embodiment by way of non-limiting examples.

In the drawings:

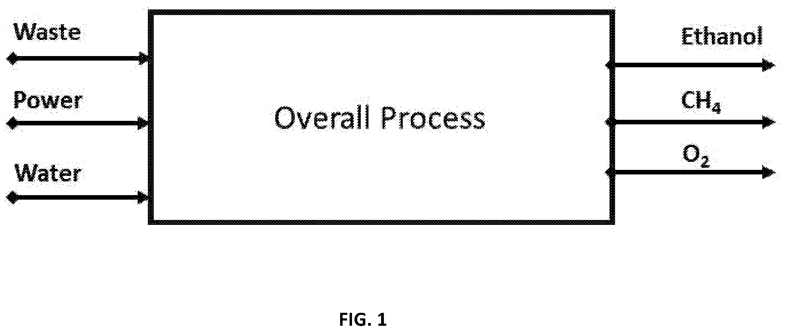

Figure 1 shows a general conceptual diagram of the invention.

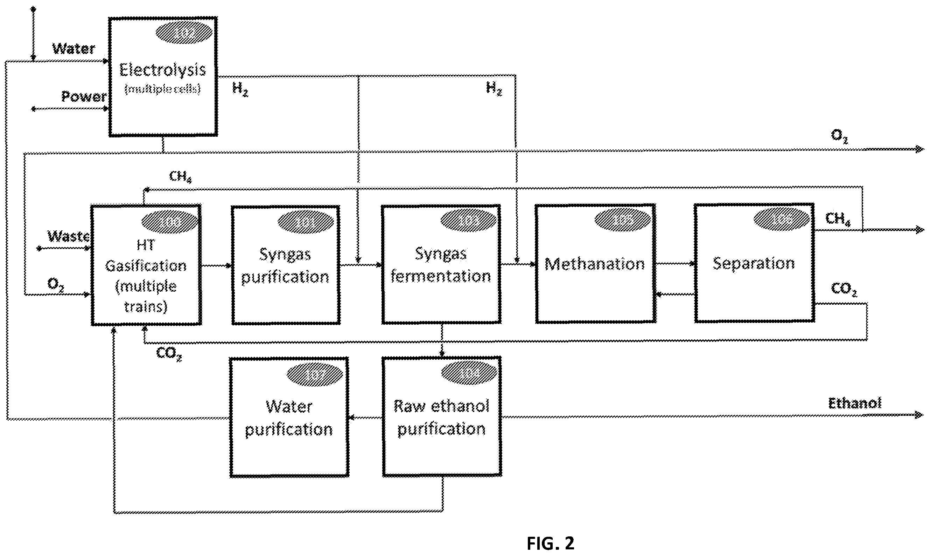

Figure 2 shows a block diagram of a preferred embodiment of the invention with introduction of hydrogen upstream and downstream of the fermenter, and with units for refining the raw ethanol and purifying the recovered water.

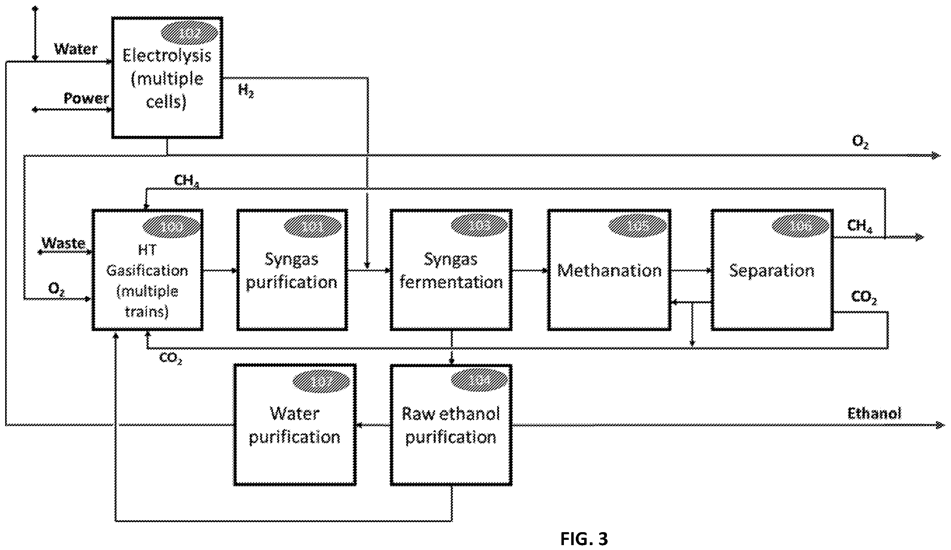

Figure 3 shows a block diagram of an alternative embodiment of the invention with introduction of hydrogen completely upstream of the fermenter.
Figure 4 shows a detailed diagram of a preferred embodiment with introduction of hydrogen upstream and downstream of the fermenter.
Figure 5 is an alternative embodiment with respect to figure 4 in which hydrogen is only introduced upstream of the fermenter.

### Detailed description of the invention

The present invention relates to a process for producing raw bioethanol by means of the fermentation of the syngas produced by the thermal conversion of the feed composed of one or more types of waste.

The invention further describes an apparatus for carrying out the aforementioned process.

The process and apparatus described allow the conversion of waste into biofuel such as, for example, ethanol; said waste comprises Municipal Solid Waste (MSW), agricultural waste or derivatives thereof such as Refuse Derived Fuel (RFD) or even industrial waste, such as non-recyclable plastic waste.

According to a first feature of the invention, the process exploits the availability of hydrogen, produced by electrolysis, to allow the conversion into ethanol of the carbonaceous elements contained in the gasified waste.

The availability of hydrogen produced by electrolysis further allows to avoid any emission of CO₂ into the atmosphere since any carbonaceous elements not converted during the fermentation step are converted into methane through a methanation reaction by simply adding further hydrogen; the methane thus produced can be sent for distribution or recycled to the gasifier to control the waste conversion temperature.

The process developed originates from the consideration that the syngas produced by the high temperature conversion of municipal solid waste, treated or not, or by the thermal conversion of agricultural or industrial waste, has a H₂/CO ratio by volume equal to about 1.

The optimal H₂/CO ratio required to produce ethanol by fermentation is about 2.0 ÷ 2.2 by volume and thus it is obvious that the thermal conversion of waste, such as the above mentioned, which produces a syngas with a lower ratio value than the optimal one, would result into an excess of carbonaceous components which would come out from the process mainly in the form of carbon dioxide.

According to another feature of the invention, it has been provided that the process comprises a plurality of high temperature waste converters able to produce the syngas and a plurality of electrolysis cells for producing hydrogen and oxygen.

By integrating the syngas produced by the converters with the hydrogen (H₂), produced by electrolysis, it is possible to use almost totally all of the carbonaceous component in the form of CO/CO₂ of the syngas for producing ethanol; furthermore, in order to maximize the CO₂ conversion , thus avoiding releasing it into the atmosphere, it is provided that the residual component, still present in the streams downstream of the fermenter, is converted into synthetic methane through the so-called methanation reaction

CO₂ + 4H₂ ←→ 2H₂O + CH₄

Advantageously, said methane can be used in the high temperature conversion of the waste for better control of the process and sent at the battery limits for use outside this process.

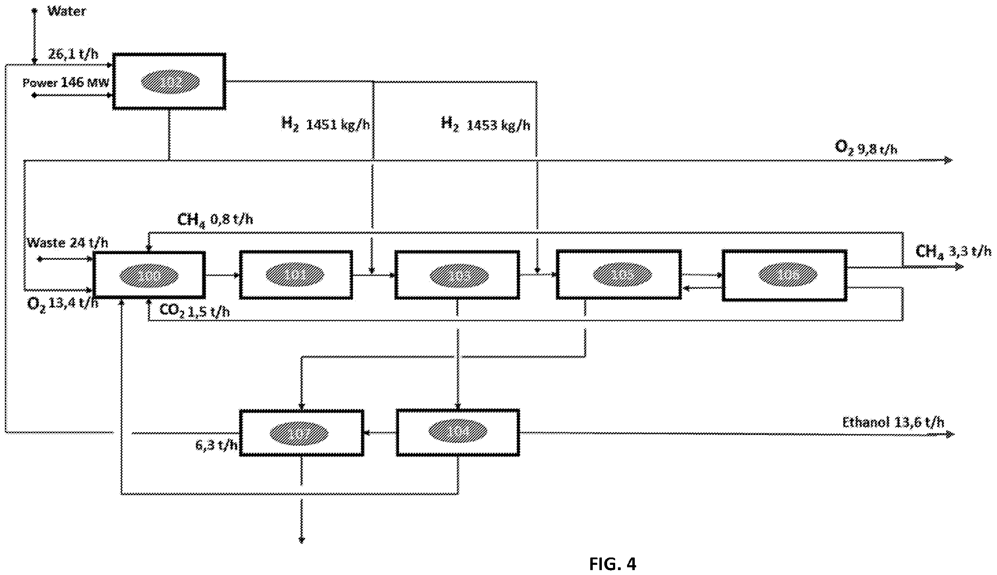

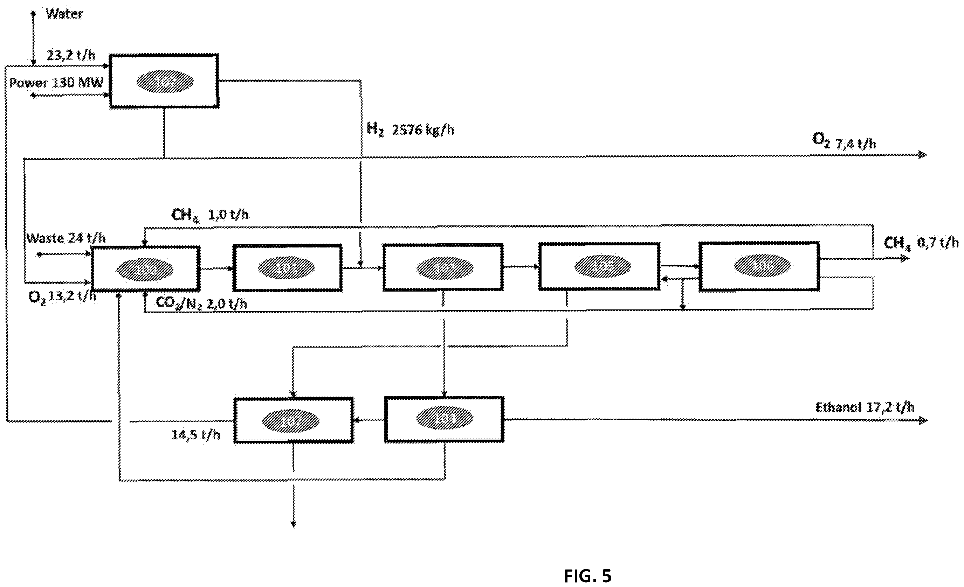

The block diagrams of the embodiments described can be seen in figures 4 and 5; alternative embodiments of the invention are however possible without altering the inventive concept underlying the invention.

Rather, the embodiments described herein aim to provide a complete and exhaustive disclosure which completely transmits the purpose of the invention to the expert skilled in the art.

Therefore, the present invention relates to an eco-compatible process for producing ethanol from waste, through a step of fermentation of the syngas, said fermentation being optimized by adding extra hydrogen obtained through electrolysis of water whereas the oxygen of the same electrolysis is used as a comburent within the thermal conversion step, allowing to avoid the emission of CO₂ into the atmosphere.

In a preferred but not limiting embodiment, such a process comprises the following main steps:
- High temperature thermal conversion of the waste with production of raw syngas (100);
- Production of H₂ and O₂ through electrolysis (102);
- Purification of the raw syngas (101);
- Fermentation (103) of the purified syngas for producing raw bioethanol, after the addition of all or part of the hydrogen produced by electrolysis for achieving the optimal H₂/CO ratio required for the fermentation reactions;
- Partial or total conversion of the residual carbon dioxide present in the purge gas from the fermentation into methane (105);
- Separation (106) of the produced methane from the unconverted carbon dioxide, and recycling of all or part of the methane recovered and all the CO₂ separated to the high temperature thermal conversion step (100);
- Purification of the raw bioethanol (104) produced by fermentation;

Said main steps can also include some further secondary steps such as:
- Purification of the water (107) obtained from the purification of the raw bioethanol and recycling of the same water to the electrolysis unit.

The secondary steps described above contribute to the optimization of the process in terms of utility consumptions.

In the preferred embodiment described, the step of high temperature conversion of waste to syngas (100) comprises multiple conversion trains, or reactors; more specifically it was chosen to use a minimum of two, preferably three, thermal conversion trains for converting the feed into syngas.

Such a solution allows to correctly manage the maintenance period so as to ensure a certain continuity of use; thereby even when a single train is stopped for maintaining the other two trains, are able to work at a greater capacity, thus ensuring a minimum turndown (75-80%) and continuous and almost constant operation for the steps downstream of the thermal conversion.

Furthermore, the operating choice to perform thermal conversion on multiple trains allows to equalize the composition of the various streams of syngas leaving each gasifier before the subsequent purification step; this implies the possibility to modulate the feed with which each gasifier is supplied in order to obtain a raw syngas that is suitable to be treated in the subsequent purification step, increasing the flexibility of the plant.

For example, a flow rate of waste between 8 and 10 t/h was chosen, which is supplied to each individual thermal conversion line for an overall total of 24-30 t/h of convertible waste.

The typical composition of the waste is shown in table 1.

**Table 1**

| **ELEMENT** | **AVERAGE COMPOSITION (BY WEIGHT)** | |
|---|---|---|
| C | 38 - 42 | %wt, wet basis |
| H | 5 - 6 | %wt, wet basis |
| N | 0.2 - 1 | %wt, wet basis |
| S | 0.2 - 1 | %wt, wet basis |
| Cl | 0.5 - 1 | %wt, wet basis |
| O | 19 - 22 | %wt, wet basis |
| HUMIDITY | 10 - 16 | %wt, wet basis |
| ASH | 15 - 18 | %wt, wet basis |

Each thermal converter is supplied with pure oxygen, produced by the electrolysis process (102), as a gasifying agent; moreover, there is provided the introduction of a certain aliquot of natural gas (CH₄), recycled from the methanation step (105) and subsequent separation (106), for the purpose of controlling the temperature profile inside the reactor.

The stream of CO₂ can also advantageously be used for inerting the waste supply system thus preventing any syngas losses and any air infiltration; in the embodiment described it was chosen to use CO₂ as an inerting agent which is likely to be available within the streams that evolve in the process.

It is however possible to use other alternative inert gases to CO₂, such as, for example, nitrogen-rich streams, for reaching the same purpose of preventing any syngas losses and any air infiltrations into the waste supply system.

For that purpose, it was chosen to add a further amount of hydrogen so as to allow a certain amount of CO₂ to be present downstream of the methanation step, separating it from the methane produced and recirculating it fully to the thermal conversion step (100) as an inerting agent.

Thereby, by performing the fermentation step (103) and the subsequent methanation step (105) so as not to fully convert CO₂, the unconverted amount of carbon dioxide is kept in circulation within the plant, thus achieving optimization and stabilization of all the processing steps.

Each conversion train consists of a conversion reactor which operates at almost atmospheric pressure (max 500 mbarg) and which dispenses syngas at a temperature of 1100-1200°C; said syngas is quickly cooled to 90°C through an evaporative quench which fixes the composition of the syngas obtained at high temperature inside the reactor avoiding triggering collateral reactions responsible for the formation of pollutants such as dioxins and furans.

The invention includes, downstream of the thermal conversion unit (100), a purification step which acts at dual pressure (101), comprising multiple units according to the capacity of the plant, the aim of which is to remove particulate, metals, chlorides, ammonia, COS and H₂S.

In the low-pressure area downstream of the quench, the syngas leaving every gasifier is at a pressure approximately to atmospheric pressure, about between 100-500 mbarg at most; said syngas is routed to an acid wash column operating at pH 1-3; these conditions allow removing any collected particles and metals from the syngas stream.

The syngas outcoming from the three conversion trains at the outlet of the acid columns is collected together and routed to a common alkaline wash column which neutralizes it by increasing the pH above 7 and reducing any corrosion phenomena on the downstream equipment.

A further purification step is carried out by means of a wet electrostatic precipitator (WEP) the aim of which is to remove the collected dust and which is possibly followed by a washing with subcooled water in a column with the aim of further reducing the dust and particulate in the syngas.

At the outlet of the low temperature washing section, a gas storage tank allows handling any flow rate and pressure fluctuations in the syngas; according to the invention the pressure at the gasometer is set at about 40 mbarg.

Before entering the high pressure purification section, the syngas is compressed up to 12 barg through a dedicated compression unit; the pressurized syngas is then sent to an adsorbent bed with the aim of remove the residual dust, particulate and heavy metals.

This operation is followed by a catalyst/adsorbent bed allowing the removal of HCl and a hydrolysis reactor allowing the conversion of COS and HCN into H₂S and NH₃, respectively.

The synthesis gas leaving from the hydrolysis reactor is sent to an Hg removal bed and to an H₂S removal system according to known technologies.

In this sense, the removal of H₂S can be carried out either through a system that allows the transformation of the H₂S into elemental sulfur and then removed as sulfur sludge, or through amine washing.

A typical composition of purified syngas is shown in table 2.

The syngas thus purified can be supplied to the ethanol fermentation section (103).

The data shown in table 2 confirm what has already been mentioned in relation to the H₂/CO ratio which is lower than the optimal ratio for the fermentation step.

**Table 2**

| **ELEMENT** | **AVERAGE COMPOSITION** | |
|---|---|---|
| H₂ | 40 - 38 | %mol |
| CO | 42 - 41 | %mol |
| CO₂ | 10 - 12 | %mol |
| N | 4 - 4.5 | %mol |
| H₂O | 4 - 4.5 | %mol |
| PRESSURE | 9 - 10 | Barg |
| TEMPERATURE | 40 - 45 | °C |

According to the invention, the further hydrogen required to obtain an optimal H₂/CO ratio is supplied by an electrolysis step (102) based on several cells, according to the capacity of the plant, suitable for the production of hydrogen and oxygen.

In a preferred but non-limiting embodiment of the invention, part of the hydrogen produced by electrolysis is added to the syngas upstream of the fermentation step (103) and the remaining part is added downstream of the fermentation unit (103), before the methanation step (105) with the aim of optimizing both steps in terms of volume and operating conditions of the equipment involved; in an alternative embodiment, the stream of hydrogen produced in the electrolysis is fully added before the fermentation step (103) promoting the maximization of the conversion of CO and CO₂ into ethanol.

In both of the solutions described, the oxygen is sent to the conversion trains to carry out the waste conversion (100).

According to the invention the conversion of CO and CO₂ into ethanol is carried out in a fermentation step (103) in which said conversion is carried out in one or more bioreactors containing a bacterial culture dispersed in a liquid nutrient medium.

As is known an organic stream consisting of single carbon atoms (C₁) and thus comprising one or more of the elements CO and CO₂, in combination with H₂, is converted into ethanol at low temperature 30-40°C and low pressure 3-5 barg.

Therefore, according to the invention, the synthesis gas containing CO, CO₂, added with the H₂ incoming from electrolysis, is bubbled inside the liquid nutrient medium; the conversion causes the formation of dispersed ethanol in the nutrient medium in the aqueous phase and the production of a residual gaseous stream containing unreacted components and CO₂ generated by the biological conversion process during fermentation.

The ethanol concentration in the stream outcoming from the fermentation step is comprised in the range between 3-6 %wt and thus it is required a specific step to separate ethanol from the aqueous phase in order to obtain anhydrous ethanol.

In a preferred embodiment the synthesis gas, in addition with part of the hydrogen produced by electrolysis and therefore having an H₂/CO ratio between 2.0 - 2.2 %vol, is converted into raw ethanol and a purge gas.

In an alternative embodiment, the syngas, in addition with all the hydrogen produced by electrolysis, will have an H₂/CO ratio between 5 - 5.2 %vol thus converting CO and CO₂ into ethanol; according to this operating mode, the CO₂ initially contained in the syngas is converted during the fermentation process with a consequent increase in the yield of ethanol and a minimization of the purge gas stream.

The purge gas produced by the fermentation step is further treated in the methanation step (105) in order to convert the CO₂ still present in the gas into methane preventing emissions into the environment.

In the preferred embodiment described, the remaining part of the hydrogen produced by electrolysis (102) is added to the stream of purge gas downstream of the fermentation step (103) in order to make the H₂/C₁ optimal for performing the methanation of the amount of CO₂ still present in the purge gas and with the aim to produce synthetic methane; in the alternative embodiment described in which the hydrogen produced by electrolysis is fully added before the fermentation step, and thus generating a minimum amount of purge gas, the remaining amount of residue H₂ in the outcoming stream of purge gas at the outlet of the fermenter is sufficient for carrying out the methanation of residual CO and CO₂ without any further addition of fresh H₂.

According to the invention the methane produced in step 105 is separated from any CO₂ unreacted in step 106, for the purpose of avoiding any emissions of CO₂ into the atmosphere.

As previously said, part of the methane is recycled to the conversion reactors (100) with the aim to control the temperature profile inside the reactors, while the remaining part is sent to other uses or sold externally.

CO₂ is recycled in part to the methanation step (105) and in part to the thermal conversion step (100) as an inerting agent for the waste supply system; in particular in the alternative embodiment described, where the hydrogen from electrolysis (102) is fully added upstream of the fermenter (103), the amount of CO₂ recovered in the separation step (106) is low and not sufficient for inerting the waste supply system.

Therefore in the alternative embodiment described, in addition to the recovered CO₂, a nitrogen-rich stream deriving from the known CO₂ separation process is used and which corresponds to a sub-product of the same block 106, as shown in fig. 5.

The liquid phase outcoming from the fermenter, containing diluted ethanol, can be sent to the raw bioethanol, produced by fermentation, purification step (104) for recovering the product; in general this step typically includes the recovery of the biocatalyst, which is recycled again to the fermenter, and an ethanol recovery and purification step, generally carried out by means of several distillation columns, followed by dehydration through molecular sieve filtration in order to comply with the requirements on the residual water.

Therefore, from the purification unit (104) a stream of anhydrous ethanol and a stream of water containing traces of alcohols produced during the fermentation process as sub-products are obtained; generally, such sub-products consist of butanediol, ethyl acetate and other higher alcohols.

Said aqueous stream produced during the distillation step can be routed to a waste water treatment step (107) such as to allow the recycling of the water purified at the electrolysis step (102).

Furthermore, according to the invention, the stream containing the sub-products consisting of butanediol, ethyl acetate and higher alcohols, and also the excess bacteria introduced into the fermenter (103), can be advantageously sent to the thermal conversion step (100); thereby, there is obtained the dual advantage of not dispersing organic components into the environment and further contributing to controlling the thermal conversion in terms of the composition of the raw syngas produced.

Advantageously, the described process allows producing bioethanol from waste such as RDF, municipal solid waste, plastic residues and the like without introducing CO₂ into the atmosphere; indeed, the synergistic effect of the fermentation step followed by the methanation step of the purge gas produced during fermentation, allows to reduce, or completely break down, the amount of CO₂ in the outlet stream, thus avoiding introduction into the atmosphere.

As mentioned in the embodiments described, the methanation step does not completely remove CO₂ but preserves a certain amount thereof which is however separated downstream of methanation, thus preventing its introduction into the atmosphere; said recovered CO₂ stream is advantageously used to inert the waste supply system, thus preventing any syngas losses and any air infiltrations which would lead to lower conversion yields of the reactor with subsequent imbalances in the fermentation steps.

## Claims

1. A process for producing ethanol by anaerobic fermentation of a synthesis gas, **characterized in that**:
• said syngas is produced by the thermal conversion at high temperature, above 1000°C, of a feed comprising municipal solid waste (MSW), agricultural waste or derivatives thereof such as refused derived fuel (RDF) or even industrial waste such as non-recyclable plastic waste or a combination thereof, and
• further hydrogen is added to said syngas, produced by electrolysis so as to balance the H₂/CO ratio to a value equal to at least 2,0 ÷ 2,2 in volume, thus maximizing the conversion of the organic components in the fermentation step so as to avoid the emission of CO₂ into the atmosphere,
and wherein
said process comprises the following steps:
• High temperature conversion of the waste with production of raw syngas (100);
• Production of H₂ and O₂ through electrolysis (102);
• Purification of the raw syngas by a purification unit which acts on two pressure levels (101), the purpose of which is the removal of particulate, metals, chlorides, ammonia, COS, and H₂S (101);
• Fermentation of the purified syngas for producing raw bioethanol, after the addition of all or part of the hydrogen produced by electrolysis for achieving the optimal H₂/CO ratio required for the fermentation reactions (103);
• Partial or total conversion of the residual carbon dioxide present in the purge gas outcoming from the fermentation into methane (105);
• Separation (106) of the methane produced by unconverted carbon dioxide, and recycling of all or part of the methane recovered and all the CO₂ separated to the thermal conversion step (100).

2. A process according to the preceding claim **characterized in that** it further includes a step (104) of purifying the raw ethanol produced by fermentation from which a stream of anhydrous ethanol and an aqueous stream containing alcohols and other organic products exit.

3. A process according to the preceding claim **characterized in that** it further includes a step of purifying the water (107) obtained in the raw ethanol purification step in order to recycle the recovered water to the electrolysis unit.

4. A process according to one or more of the preceding claims **characterized in that** the high temperature waste conversion step for the production of raw syngas (100) consists of a plurality of converters, providing at least of two trains thereof, preferably three conversion trains.

5. A process according to one or more of the preceding claims **characterized in that** the step of purifying the syngas produced (101) consists of a plurality of trains depending on the capacity of the plant.

6. A process according to one or more of the preceding claims **characterized in that**, in the water electrolysis step, several cells are used depending on the capacity of the plant.

7. A process according to one or more of claims from 1 to 6 **characterized in that** the hydrogen produced in the electrolysis step (102) is partially added upstream of the fermentation step, thus shifting the H₂/CO ratio to a value between 2.0 and 2.2 %vol, and the remaining part is added downstream of the fermentation step in order to enable the development of methanation reactions (105), thus optimizing the process in terms of volumes and operating conditions of the fermentation and methanation steps.

8. A process according to one or more of claims from 1 to 6 **characterized in that** the hydrogen produced in the electrolysis step (102) is fully added upstream of the fermentation step, thus shifting the H₂/CO ratio to a value between 5 and 5.2 %vol, maximizing the conversion process of CO and CO₂ into bioethanol.

9. A process according to one or more of the preceding claims **characterized in that** the oxygen produced by electrolysis is used as a combustive agent in the conversion step for converting waste into syngas.

10. A process according to one or more of the preceding claims **characterized in that** the fermentation step (103) is carried out in one or more bioreactors, depending on the capacity of the plant, containing a bacterial culture dispersed in a liquid nutrient medium.

11. A process according to one or more of the preceding claims **characterized in that** an amount of the methane stream produced during methanation (105) and exiting from the separation step (106) is recycled to the thermal waste conversion step (100) in order to control the conversion temperature of the feed.

12. A process according to one or more of the preceding claims **characterized in that** the excess bacteria exiting from the fermentation step (103) and the byproducts consisting of butanediol, ethyl acetate, and superior alcohols exiting from the water purification unit (107), are routed to the converter in order to control the conversion of waste in terms of composition of the syngas produced.

13. A process according to one or more of the preceding claims **characterized in that** the CO₂ outcoming from the separation process (106) is used individually or in a mixture with other streams, consisting of inert substances and which form byproducts of the same separation step (106), for inerting the waste supply system thus preventing any syngas losses and any air infiltration, and allowing an equalization of the conversion yields.

14. An apparatus for producing ethanol by anaerobic fermentation of a synthesis gas **characterized in that** it includes the conversion into said ethanol of the syngas produced by the thermal conversion of municipal solid waste (MSW), agricultural waste or derivatives thereof such as refused derived fuel (RFD) or even industrial waste such as non-recyclable plastic waste, wherein said process does not imply any CO₂ emission into the atmosphere as it uses electrolysis for the production of further hydrogen to be added to said syngas so as to balance the H₂/CO ratio, thus maximizing the conversion of the organic components in the fermentation step,
and wherein
said apparatus comprises the following steps:
• at least one thermal, high temperature waste conversion unit with the production of raw syngas (100) consisting of a plurality of conversion reactors;
• at least one unit for producing H₂ and O₂ by electrolysis (102) consisting of a plurality of electrolysis cells;
• at least one raw syngas purification unit consisting of a plurality of purification trains (101) acting at double stage pressure, low pressure and high pressure, the purpose of said purification trains (101) being to remove particulate, metals, chlorides, ammonia, COS, and H₂S;
• at least one unit for the fermentation of the purified syngas, consisting of a plurality of bioreactors, for producing raw bioethanol, upon the addition of all or part of the hydrogen produced by electrolysis for achieving the optimal H₂/CO ratio required for the fermentation reactions (103);
• at least one unit for the partial or total conversion of the residual carbon dioxide present in the purge gas from the fermentation into methane (105);
• at least one separation unit (106) for separating the produced methane from unconverted carbon dioxide and a line for recycling both of the streams to the converter;
• at least one recycling line exiting from the separation step (106) and routed to the thermal conversion step (100) for recycling all or part of the methane, exiting from the separation step, as a temperature control vector in the thermal conversion step;
• at least one recycling line exiting from the separation step (106) and routed to the thermal conversion step (100) for recycling all of the CO₂, recovered in the separation step, as an inerting agent in the thermal conversion step.

15. An apparatus according to claim 14 **characterized in that** it further includes at least one purification unit (104) for purifying the raw ethanol produced by fermentation from which a stream of anhydrous ethanol and an aqueous stream containing alcohols and other organic products exit.

16. An apparatus according to claims 14 and 15 **characterized in that** it further includes at least one water purification unit (107) for the aqueous stream produced in the raw ethanol purification step in order to recycle the recovered water to the electrolysis unit.

## Patentansprüche

1. Verfahren zur Herstellung von Ethanol durch anaerobe Fermentation eines Syngases , **dadurch gekennzeichnet, dass**:
• das genannte Syngas durch thermische Umwandlung eines Einsatzmaterials, das feste Siedlungsabfälle (MSW), landwirtschaftliche Abfälle oder Derivate davon, wie beispielsweise Ersatzbrennstoffe (RDF), oder sogar Industrieabfälle, wie beispielsweise nicht wiederverwertbare Kunststoffabfälle, oder eine Kombination davon umfasst, bei hoher Temperatur von über 1000 °C hergestellt wird; und
• dem genannten Syngas durch Elektrolyse erzeugter weiterer Wasserstoff zugesetzt wird, um das H₂/CO-Verhältnis auf einen Wert von mindestens 2,0 ÷ 2,2 nach Volumen auszugleichen, wodurch die Umwandlung der organischen Bestandteile in dem Fermentationsschritt maximiert wird, um die Freisetzung von CO₂in die Atmosphäre zu vermeiden;
und wobei
das genannte Verfahren die folgenden Schritte umfasst:
• Hochtemperaturumwandlung des Abfalls mit Erzeugung von Roh-Syngas (100);
• Erzeugen von H₂ und O₂ durch Elektrolyse (102);
• Reinigen des rohen Syngases durch eine auf zwei Druckstufen wirkende Reinigungseinheit (101), deren Zweck das Entfernen von Partikeln, Metallen, Chloriden, Ammoniak, COS und H₂S ist (101);
• Fermentation des gereinigten Syngases zur Herstellung von Roh-Bioethanol nach Zugabe des gesamten oder eines Teils des durch Elektrolyse erzeugten Wasserstoffs, um das für die Fermentationsreaktionen erforderliche optimale H₂/CO-Verhältnis zu erreichen (103);
• Teilweises oder vollständiges Umwandeln des in dem aus der Fermentation austretenden Spülgas vorhandenen Restkohlendioxids in Methan (105);
• Abtrennen (106) des erzeugten Methans von dem nicht umgewandelten Kohlendioxid und Rückführen des gesamten oder eines Teils des wiedergewonnenen Methans und des gesamten abgetrennten CO₂ in den thermischen Umwandlungsschritt (100);

2. Verfahren nach dem vorhergehenden Anspruch, **dadurch gekennzeichnet, dass** es ferner einen Schritt (104) des Reinigens des durch Fermentation erzeugten Rohethanols umfasst, aus dem ein wasserfreier Ethanolstrom und ein wässriger Strom, der Alkohole und andere organische Produkte enthält, austreten.

3. Verfahren nach dem vorhergehenden Anspruch, **dadurch gekennzeichnet, dass** es ferner einen Schritt des Reinigens des im Reinigungsschritt des Rohethanols erhaltenen Wassers (107) umfasst, um das wiedergewonnene Wasser in die Elektrolyseeinheit rückzuführen.

4. Verfahren nach einem oder mehreren der vorhergehenden Ansprüche, **dadurch gekennzeichnet, dass** der Hochtemperatur-Abfallumwandlungsschritt zur Herstellung von Roh-Syngas (100) aus einer Vielzahl von Konvertern besteht, die mindestens zwei, vorzugsweise drei Umwandlungsstränge bilden.

5. Verfahren nach einem oder mehreren der vorhergehenden Ansprüche, **dadurch gekennzeichnet, dass** der Schritt des Reinigens des erzeugten Syngases (101) aus einer Vielzahl von Strängen in Abhängigkeit von der Kapazität der Anlage besteht.

6. Verfahren nach einem oder mehreren der vorhergehenden Ansprüche, **dadurch gekennzeichnet, dass** im Schritt der Wasserelektrolyse mehrere Zellen in Abhängigkeit von der Kapazität der Anlage verwendet werden.

7. Verfahren nach einem oder mehreren der Ansprüche 1 bis 6, **dadurch gekennzeichnet, dass** der in dem Schritt der Elektrolyse (102) erzeugte Wasserstoff teilweise stromaufwärts des Fermentationsschrittes zugegeben wird, wodurch das H₂/CO-Verhältnis auf einen Wert zwischen 2,0 und 2,2 Vol.-% verschoben wird, und der verbleibende Teil stromabwärts des Fermentationsschrittes zugegeben wird, um zu bewirken, dass sich Methanisierungsreaktionen (105) entwickeln, wodurch das Verfahren in Bezug auf die Volumina und die Betriebsbedingungen der Schritte der Fermentation und der Methanisierung optimiert wird.

8. Verfahren nach einem oder mehreren der Ansprüche 1 bis 6, **dadurch gekennzeichnet, dass** der in dem Schritt der Elektrolyse (102) erzeugte Wasserstoff vollständig stromaufwärts des Fermentationsschrittes zugegeben wird, wodurch das H₂/CO-Verhältnis auf einen Wert zwischen 5 und 5,2 Vol.-% verschoben wird, wodurch der Umwandlungsprozess von CO und CO₂ in Bioethanol maximiert wird.

9. Verfahren nach einem oder mehreren der vorhergehenden Ansprüche, **dadurch gekennzeichnet, dass** der durch Elektrolyse erzeugte Sauerstoff als Verbrennungsmittel in dem Umwandlungsschritt zur Umwandlung von Abfall in Syngas verwendet wird.

10. Verfahren nach einem oder mehreren der vorhergehenden Ansprüche, **dadurch gekennzeichnet, dass** der Fermentationsschritt (103) in einem oder mehreren Bioreaktoren, je nach Kapazität der Anlage, durchgeführt wird, die ein in einem flüssigen Nährmedium dispergiertes Bakterienwachstum enthalten.

11. Verfahren nach einem oder mehreren der vorhergehenden Ansprüche, **dadurch gekennzeichnet, dass** eine Menge des während der Methanisierung (105) erzeugten und aus dem Abtrennungsschritt (106) austretenden Methanstroms in den thermischen Abfallumwandlungsschritt (100) rückgeführt wird, um die Umwandlungstemperatur des Einsatzmaterials zu steuern.

12. Verfahren nach einem oder mehreren der vorhergehenden Ansprüche, **dadurch gekennzeichnet, dass** die aus dem Fermentationsschritt (103) austretenden überschüssigen Bakterien und die aus Butandiol, Ethylacetat und höheren Alkoholen bestehenden Nebenprodukte, die aus der Wasserreinigungseinheit (107) austreten, dem Konverter zugeführt werden, um die Umwandlung der Abfälle in Bezug auf die Zusammensetzung des erzeugten Syngases zu steuern.

13. Verfahren nach einem oder mehreren der vorhergehenden Ansprüche, **dadurch gekennzeichnet, dass** das aus dem Abtrennungsprozess (106) austretende CO₂ einzeln oder in einem Gemisch mit anderen Strömen, die aus inerten Substanzen bestehen und die Nebenprodukte desselben Abtrennungsschritts (106) bilden, zur Inertisierung des Abfallversorgungssystems verwendet wird, wodurch jegliche Syngasverluste und jegliches Eindringen von Luft verhindert werden und ein Ausgleich der Umwandlungsausbeuten ermöglicht wird.

14. Vorrichtung zur Herstellung von Ethanol durch anaerobe Fermentation eines Syngases, **dadurch gekennzeichnet, dass** sie die Umwandlung des genannten Syngases, das durch die thermische Umwandlung von festen Siedlungsabfällen (MSW), landwirtschaftlichen Abfällen oder Derivaten davon, wie beispielsweise Ersatzbrennstoffe (RFD), oder sogar Industrieabfällen, wie beispielsweise nicht wiederverwertbare Kunststoffabfälle, hergestellt wurde, in das genannte Ethanol umfasst, wobei das genannte Verfahren keine CO₂-Emission in die Atmosphäre impliziert, da es die Elektrolyse zur Erzeugung von weiterem Wasserstoff verwendet, der dem genannten Syngas zugesetzt wird, um das H₂/CO-Verhältnis auszugleichen, wodurch die Umwandlung der organischen Verbindungen in dem Fermentationsschritt maximiert wird;
und wobei
die genannte Vorrichtung die folgenden Schritte umfasst:
• mindestens eine aus einer Vielzahl von Konversionsreaktoren bestehende thermische Hochtemperatur-Abfallkonversionsanlage mit Erzeugung von Roh-Syngas (100);
• mindestens eine aus einer Vielzahl von Elektrolysezellen bestehende Einheit zur Herstellung von H₂ und O₂ durch Elektrolyse (102);
• mindestens eine aus einer Vielzahl von Reinigungssträngen (101) bestehende Einheit zur Reinigung des Roh-Syngases, die mit zweistufigem Druck, Niederdruck und Hochdruck arbeiten, wobei der Zweck der genannten Reinigungsstränge (101) darin besteht, Partikel, Metalle, Chloride, Ammoniak, COS und H₂S zu entfernen;
• mindestens eine aus einer Vielzahl von Bioreaktoren bestehende Einheit für die Fermentation des gereinigten Syngases zur Herstellung von rohem Bioethanol unter Zugabe des gesamten oder eines Teils des durch Elektrolyse erzeugten Wasserstoffs, um das für die Fermentationsreaktionen erforderliche optimale H₂/CO-Verhältnis zu erreichen (103);
• mindestens eine Einheit zur teilweisen oder vollständigen Umwandlung des im Spülgas der Fermentation vorhandenen Restkohlendioxids in Methan (105);
• mindestens eine Trenneinheit (106) zur Abtrennung des erzeugten Methans vom nicht umgewandelten Kohlendioxid und eine Leitung zur Rückführung beider Ströme zum Konverter;
• mindestens eine aus dem Abtrennungsschritt (106) austretende und zum thermischen Umwandlungsschritt (100) geführte Rückführungsleitung zur Rückführung des gesamten oder eines Teils des aus dem Abtrennungsschritt austretenden Methans als Temperaturregelvektor im thermischen Umwandlungsschritt;
• mindestens eine aus dem Abtrennungsschritt (106) austretende und zum thermischen Umwandlungsschritt (100) geführte Rückführungsleitung zur Rückführung des gesamten, im Abtrennungsschritt wiedergewonnenen CO₂ als Inertisierungsmittel im thermischen Umwandlungsschritt.

15. Vorrichtung nach Anspruch 14, **dadurch gekennzeichnet, dass** sie ferner mindestens eine Reinigungseinheit (104) zum Reinigen des durch Fermentation erzeugten Rohethanols umfasst, aus der ein Strom wasserfreien Ethanols und ein Alkohole und andere organische Produkte enthaltender wässriger Strom austreten.

16. Vorrichtung nach einem der Ansprüche 14 und 15, **dadurch gekennzeichnet, dass** sie ferner mindestens eine Wasserreinigungseinheit (107) für den wässrigen Strom umfasst, der im Reinigungsschritt für Rohethanol erzeugt wird, um das wiedergewonnene Wasser in die Elektrolyseeinheit rückzuführen.

## Revendications

1. Procédé de production d'éthanol par fermentation anaérobie d'un gaz de synthèse, **caractérisé en ce que** :
• ledit gaz de synthèse est produit par la conversion thermique à haute température, supérieure à 1000 °C, d'une charge d'alimentation comprenant des déchets ménagers (DMS), des déchets agricoles ou des dérivés de ceux-ci tels qu'un combustible dérivé de déchets (DFD) ou même des déchets industriels tels que des déchets plastiques non recyclables ou une combinaison de ceux-ci, et
• de l'hydrogène supplémentaire est ajouté audit gaz de synthèse, produit par électrolyse de manière à équilibrer le rapport H₂/CO à une valeur égale à au moins 2,0 ÷ 2,2 en volume, pour ainsi maximiser la conversion des composants organiques dans l'étape de fermentation de manière à éviter l'émission de CO₂ dans l'atmosphère,
et dans lequel
ledit procédé comprend les étapes suivantes :
• la conversion à haute température des déchets avec production d'un gaz de synthèse brut (100) ;
• la production de H₂ et d'O₂ par électrolyse (102) ;
• la purification du gaz de synthèse brut par une unité de purification qui agit sur deux niveaux de pression (101), le but étant d'éliminer les particules, les métaux, les chlorures, l'ammoniac, le COS et le H₂S (101) ;
• la fermentation du gaz de synthèse purifié pour produire du bioéthanol brut, après ajout de tout ou partie de l'hydrogène produit par électrolyse pour obtenir le rapport H₂/CO optimal requis pour les réactions de fermentation (103) ;
• la conversion partielle ou totale du dioxyde de carbone résiduel présent dans le gaz de purge issu de la fermentation en méthane (105) ;
• la séparation (106) du méthane produit du dioxyde de carbone non converti, et le recyclage de tout ou partie du méthane récupéré et de la totalité du CO₂ séparé vers l'étape de conversion thermique (100).

2. Procédé selon la revendication précédente **caractérisé en ce qu'**il comporte en outre une étape (104) de purification de l'éthanol brut produit par fermentation de laquelle sortent un courant d'éthanol anhydre et un courant aqueux contenant des alcools et d'autres produits organiques.

3. Procédé selon la revendication précédente **caractérisé en ce qu'**il comporte en outre une étape de purification de l'eau (107) obtenue dans l'étape de purification de l'éthanol brut afin de recycler l'eau récupérée vers l'unité d'électrolyse.

4. Procédé selon une ou plusieurs des revendications précédentes **caractérisé en ce que** l'étape de conversion des déchets à haute température pour la production d'un gaz de synthèse brut (100) consiste en une pluralité de convertisseurs, fournissant au moins deux trains de ceux-ci, de préférence trois trains de conversion.

5. Procédé selon une ou plusieurs des revendications précédentes **caractérisé en ce que** l'étape de purification du gaz de synthèse produit (101) consiste en une pluralité de trains en fonction de la capacité de l'installation.

6. Procédé selon une ou plusieurs des revendications précédentes **caractérisé en ce que**, dans l'étape d'électrolyse de l'eau, plusieurs cellules sont utilisées en fonction de la capacité de l'installation.

7. Procédé selon une ou plusieurs des revendications 1 à 6 **caractérisé en ce que** l'hydrogène produit dans l'étape d'électrolyse (102) est partiellement ajouté en amont de l'étape de fermentation, pour ainsi déplacer le rapport H₂/CO vers une valeur comprise entre 2,0 et 2,2 % en volume, et la partie restante est ajoutée en aval de l'étape de fermentation afin de permettre le développement de réactions de méthanisation (105), pour ainsi optimiser le procédé en matière de volumes et de conditions de fonctionnement des étapes de fermentation et de méthanisation.

8. Procédé selon une ou plusieurs des revendications 1 à 6 **caractérisé en ce que** l'hydrogène produit dans l'étape d'électrolyse (102) est entièrement ajouté en amont de l'étape de fermentation, pour ainsi déplacer le rapport H₂/CO vers une valeur comprise entre 5 et 5,2 % en volume, pour maximiser le procédé de conversion du CO et du CO₂ en bioéthanol.

9. Procédé selon une ou plusieurs des revendications précédentes **caractérisé en ce que** l'oxygène produit par électrolyse est utilisé comme agent combustible dans l'étape de conversion pour convertir les déchets en gaz de synthèse.

10. Procédé selon une ou plusieurs des revendications précédentes **caractérisé en ce que** l'étape de fermentation (103) est réalisée dans un ou plusieurs bioréacteurs, en fonction de la capacité de l'installation, contenant une culture bactérienne dispersée dans un milieu nutritif liquide.

11. Procédé selon une ou plusieurs des revendications précédentes **caractérisé en ce qu'**une quantité du courant de méthane produit pendant la méthanisation (105) et sortant de l'étape de séparation (106) est recyclée vers l'étape de conversion thermique des déchets (100) afin de réguler la température de conversion de la charge d'alimentation.

12. Procédé selon une ou plusieurs des revendications précédentes **caractérisé en ce que** les bactéries en excès sortant de l'étape de fermentation (103) et les sous-produits consistant en du butanediol, de l'acétate d'éthyle et des alcools supérieurs sortant de l'unité de purification d'eau (107), sont acheminés vers le convertisseur afin de contrôler la conversion des déchets en matière de composition du gaz de synthèse produit.

13. Procédé selon une ou plusieurs des revendications précédentes **caractérisé en ce que** le CO₂ issu du procédé de séparation (106) est utilisé individuellement ou en mélange avec d'autres courants, consistant en des substances inertes et qui forment des sous-produits de la même étape de séparation (106), pour rendre inerte le système d'alimentation en déchets, pour ainsi éviter toute perte de gaz de synthèse et toute infiltration d'air, et permettre une égalisation des rendements de conversion.

14. Appareil de production d'éthanol par fermentation anaérobie d'un gaz de synthèse, **caractérisée en ce qu'**elle comporte la conversion, en ledit éthanol, du gaz de synthèse produit par la conversion thermique de déchets ménagers (DMS), de déchets agricoles ou de dérivés de ceux-ci tels qu'un combustible dérivé de déchets (DFD) ou même de déchets industriels tels que des déchets plastiques non recyclables, dans lequel ledit procédé n'implique aucune émission de CO₂ dans l'atmosphère car il utilise une électrolyse pour la production d'hydrogène supplémentaire à ajouter audit gaz de synthèse de manière à équilibrer le rapport H₂/CO, pour ainsi maximiser la conversion des composants organiques dans l'étape de fermentation,
et dans lequel
ledit appareil comprend les étapes suivantes :
• au moins une unité de conversion thermique de déchets à haute température avec la production d'un gaz de synthèse brut (100) constituée d'une pluralité de réacteurs de conversion ;
• au moins une unité de production de H₂ et de O₂ par électrolyse (102) constituée d'une pluralité de cellules d'électrolyse ;
• au moins une unité de purification de gaz de synthèse brut constituée d'une pluralité de trains de purification (101) agissant à une double pression d'étage, basse pression et haute pression, le but desdits trains de purification (101) étant d'éliminer les particules, les métaux, les chlorures, l'ammoniac, le COS et le H₂S ;
• au moins une unité pour la fermentation du gaz de synthèse purifié, constituée d'une pluralité de bioréacteurs, pour produire du bioéthanol brut, après ajout de tout ou partie de l'hydrogène produit par électrolyse pour obtenir le rapport H₂/CO optimal requis pour les réactions de fermentation (103) ;
• au moins une unité pour la conversion partielle ou totale du dioxyde de carbone résiduel présent dans le gaz de purge de la fermentation en méthane (105) ;
• au moins une unité de séparation (106) pour séparer le méthane produit du dioxyde de carbone non converti et une conduite pour recycler des deux courants vers le convertisseur ;
• au moins une conduite de recyclage sortant de l'étape de séparation (106) et allant vers l'étape de conversion thermique (100) pour le recyclage de tout ou partie du méthane, sortant de l'étape de séparation, en tant que vecteur de régulation de température dans l'étape de conversion thermique ;
• au moins une conduite de recyclage sortant de l'étape de séparation (106) et allant vers l'étape de conversion thermique (100) pour le recyclage de la totalité du CO₂, récupéré dans l'étape de séparation, en tant qu'agent d'inertage dans l'étape de conversion thermique.

15. Appareil selon la revendication 14 **caractérisé en ce qu'**il comporte en outre au moins une unité de purification (104) pour purifier l'éthanol brut produit par fermentation de laquelle sortent un courant d'éthanol anhydre et un courant aqueux contenant des alcools et d'autres produits organiques.

16. Appareil selon les revendications 14 et 15 **caractérisé en ce qu'**il comporte en outre au moins une unité de purification d'eau (107) pour le courant aqueux produit dans l'étape de purification de l'éthanol brut afin de recycler l'eau récupérée vers l'unité d'électrolyse.
